# EUROPEAN PATENT APPLICATION

(11) **EP 3 656 867 A1**
(43) Date of publication of application: **27.05.2020**
(21) Application number: 18834563.1
(22) Date of filing: 17.07.2018
(51) Int. Cl.: C12Q 1/02, A61Q 19/08, C12Q 1/68, G01N 33/15

(54) **METHOD FOR SCREENING ANTI-AGING SUBSTANCES**

(30) Priority: 18.07.2017 JP 2017139181
(71) Applicant: Shiseido Company, Ltd., Tokyo 104-0061 (JP)
(72) Inventor: EZURE, Tomonobu, Yokohama-shi Kanagawa 224-8558 (JP); TOYODA, Misato, Yokohama-shi Kanagawa 224-8558 (JP)
(74) Representative: Santarelli
(86) International application number: PCT/JP2018/026783
(87) International publication number: WO 2019/017356

(57) **Abstract**

The purpose of the invention is to develop a method for screening anti-aging substances. The present inventors have found a phenomenon in which a dermis aging factor secreted by senescent cells acts on normal cells to promote the decomposition of dermal components and a reduction in the production of dermal components, and have arrived at developing a method for screening anti-aging substances by employing the dermis aging factor as an indicator.

## Description

### Technical Field

The present invention relates to a screening method for an anti-aging substance using the expression of a dermal senescence factor as an indicator.

### Background Art

Although the various symptoms associated with aging are an unavoidable problem but researches that scientifically analyze the aging to inhibit the progression of aging or ameliorate aging have been carried out. While aging is macroscopically a major factor in skin aging, which is of utmost interest in terms of beauty, there are additional direct factors that affect skin aging such as the effects of dryness, oxidation, UV, and such. The symptoms of skin aging can be seen as skin wrinkles (large wrinkles and fine wrinkles), sagging, spots, dullness, worsening of skin texture, etc. and the causes thereof include a reduction of collagen, cross-linking reactions in collagen, a decrease in mucopolysaccharides including hyaluronic acid, an increase in melanin pigment, and a decrease in epidermal turnover. These causes can be broadly divided into dermal and epidermal causes which each need to be addressed. For dermal causes, it has been elucidated that the promotion of hyaluronic acid production, inhibition of matrix metalloproteinase (MMP) production and activity, promotion of collagen production, and inhibition of esterase activity are effective for dermal anti-aging. In such anti-aging research, activation of cells from the epidermis or dermis layer has been performed, but recently, a technique for skin anti-aging by the activation of stem cells, specifically somatic stem cells, has been examined.

Stem cells are defined as cells that have the capacity to self-replicate and the capacity to differentiate into other types of cell. As the stem cells, other than pluripotent stem cells that can differentiate into all cell types that form one individual such as embryonic stem cells (ES cells) or induced pluripotent stem cells (iPS cells), multipotent somatic stem cells that are not pluripotent but can differentiate into several cell lineages are known to exist in each tissue even in an adult. It is known that somatic stem cells contribute to tissue repair at the time of tissue damage, maintenance of tissue homeostasis and aging. Somatic stem cells are known to exist in various tissues. As stem cells present in skin tissue, epidermal stem cells present in the epidermis layer, and mesenchymal stem cells present in the dermis layer and subcutaneous fat layer are known.

In the epidermis, it is well known that epidermal stem cells are present in the basal layer of the epidermis, and hair follicle epithelial stem cells and skin pigment stem cells have been reported to be present in a region called the bulge region of the hair follicle. Meanwhile, the dermis contains skin-derived precursor cells (SKP cells) that differentiate into a plurality of cell lines such as adipose, glia, cartilage, and muscle (NPL 1: Wong C. E. et al., J Cell. Biol. 175: 1005-1015, 2006). Furthermore, cells referred to as dermal mesenchymal stem cells have been isolated (PTL 1: WO2011/034106).

It is known that mesenchymal stem cells are attracted to a trauma site when the skin is subjected to trauma. It is thought that the mesenchymal stem cells that are attracted to the trauma site activate surrounding skin cells and promote cell proliferation, differentiate into skin cells, reduce the trauma, and contribute to healing. It has been shown that certain flavonoids have an effect of attracting mesenchymal stem cells (NPL 2: PLOS ONE DOI: 10.137/journal.pone.0144166). Mesenchymal stem cells that contribute to healing of skin trauma are considered to include bone marrow-derived mesenchymal stem cells which are transported through blood vessels as well as adipose stem cells that exist in the subcutaneous tissue and dermal mesenchymal stem cells that exist around organs such as sebaceous glands, sweat glands, and hair follicles and near blood vessels. Although bone marrow-derived stem cells have conventionally been the subject of research into mesenchymal stem cells, once adipose stem cells are isolated from adipose tissue, due to the availability and multipotent capacity thereof of being able to differentiate into various cell lines, the application thereof to various regenerative treatments is expected (NPL 3: World J Stem Cells 2014 January 26; 6 (1): 65-68). Dermal mesenchymal stem cells exist close to blood vessels but there are still many unanswered questions regarding the differentiative capacity and properties thereof and research is expected to be carried out henceforth regarding the application thereof.

### [CITATION LIST]

### [PATENT LITERATURE]

[PTL 1] WO2011/034106
[PTL 2] Japanese Unexamined Patent Publication (Kohyo) No. 2013-507956

### [NON-PATENT LITERATURE]

[NPL 1] J Cell Biol. 175:1005-1015, 2006
[NPL 2] PLOS ONE DOI:10.137/journal.pone.0144166
[NPL 3] World J Stem Cells 2014 January 26; 6(1): 65-68

### SUMMARY

### [TECHNICAL PROBLEM]

The object of the present invention is the development of a screening method for an anti-aging substance.

### [SOLUTION TO PROBLEM]

The present inventors conducted research on aging of the dermis and discovered a surprising phenomenon whereby a factor secreted by senescent cells (hereinafter referred to as dermal senescence factor) acted on normal cells such that the production of dermal components decreased and the degradation of dermal components was promoted. Based on these findings, the present inventors developed a screening method for an anti-aging substance using a dermal senescence factor as an indicator. More specifically, the present invention relates to the following.
[1] A screening method for an anti-aging substance using the expression of a dermal senescence factor as an indicator.
[2] The screening method according to item 1, comprising the steps of:
   culturing a skin cell culture containing a dermal fibroblast in a culture medium containing a candidate drug;
   measuring expression of the dermal senescence factor in the culture; and
   selecting a candidate drug that can inhibit the expression of the dermal senescence factor.
[3] The screening method according to item 2, wherein the skin cell culture further contains a stem cell.
[4] The screening method according to any one of items 1 to 3, wherein the expression of the dermal senescence factor is determined by measuring the gene expression or protein level of the dermal senescence factor.
[5] The screening method according to any one of items 1 to 4, wherein the dermal senescence factor is IGFBP7, CFD, or RSPO4.
[6] A dermal senescence factor expression inhibitor comprising a turmeric extract or a Rosa multiflora extract.
[7] The dermal senescence factor expression inhibitor according to item 6, wherein the dermal senescence factor is CFD.
[8] A turmeric extract or a Rosa multiflora extract for use in the treatment of wrinkles, sagging, or skin aging by expression inhibition of the dermal senescence factor.
[9] A method of administering a composition comprising a turmeric extract or a Rosa multiflora extract as an aesthetic or non-therapeutic method of ameliorating wrinkles, sagging, or skin aging.
[10] The method according to item 9, comprising ameliorating wrinkles, sagging, or skin aging through expression inhibition of a dermal senescence factor.
[12] The method according to item 9 or 10, comprising administration to a subject suffering from wrinkles, sagging, or skin aging.
[13] A use of a turmeric extract or a Rosa multiflora extract for producing an expression inhibitor of a dermal senescence factor.
[14] A skin amelioration method comprising the administration of a composition comprising a turmeric extract or a Rosa multiflora extract to a subject suffering from wrinkles, sagging, or skin aging.
[15] An aesthetic or non-therapeutic method of ameliorating wrinkles, sagging, or skin aging characterized by expression inhibition of a dermal senescence factor.
[16] The aesthetic or non-therapeutic method according to item 15, wherein the dermal senescence factor is IGFBP7, CFD, or RSPO4.
[17] The aesthetic or non-therapeutic method according to item 16, comprising the administration of a turmeric extract or a Rosa multiflora extract for expression inhibition of the dermal senescence factor.

### [ADVANTAGEOUS EFFECTS OF INVENTION]

The screening method of the present invention can screen for an anti-aging substance using a dermal senescence factor as an indicator. By elucidating a novel mechanism of aging through a dermal senescence factor and using such a factor as an indicator, it will be possible to obtain anti-aging substances that could not be screened for with conventional screening methods.

### BRIEF DESCRIPTION OF DRAWINGS

FIG. 1 (A) shows a schematic diagram of a transwell system. Normal (young) fibroblasts are cultured on the upper layer and senescent fibroblasts are cultured on the lower layer. FIG. 1(B) to (D) are graphs that compare the gene expression levels of (B) collagen 1A1 (COL1a1), (C) elastin (ELN), and (D) metalloproteinase 1 (MMP1) between (Blank) in which cells are not cultured on the lower layer and (+Senescent) in which senescent fibroblasts are cultured on the lower layer.
FIG. 2(A) is a graph showing the inhibition of CFD gene expression by senescent fibroblasts in the lower layer of the transwell system and FIG. 2(B) is a graph showing a change in MMP1 gene expression in fibroblasts in the upper layer in such cases.
FIG. 3 shows graphs comparing gene expression levels of the senescence factors (A) IGFBP7, (B) RSPO4, and (C) CFD between senescent fibroblasts and normal (young) fibroblasts.
FIG. 4 shows graphs illustrating (A) dose-dependent increase in expression of the MMP1 gene and (B) a decrease in expression of the Collagen 1 gene when IGFBP7and RSPO4 are added.
FIG. 5 shows a graph illustrating a decrease in expression of the senescence factor (IGFBP7) in the presence of mesenchymal stem cells in a co-culture system of senescent fibroblasts and mesenchymal stem cells.
FIG. 6 shows a graph illustrating a decrease in expression of the senescence factor RSPO4 compared to a control when a turmeric extract or a Rosa multiflora extract is added to a senescent fibroblast culture then cultured.

### DESCRIPTION OF EMBODIMENTS

The present invention relates to a screening method for an anti-aging substance using the expression of a dermal senescence factor as an indicator. More specifically, this screening method comprises the following steps:
culturing a skin cell culture comprising a dermal fibroblast in a culture medium comprising a candidate drug;
measuring expression of the dermal senescence factor in the culture; and
selecting a candidate drug that can inhibit the expression of the dermal senescence factor.

The screening method according to the present invention may further comprise a preculturing step of culturing a skin cell culture comprising a dermal fibroblast in a culture medium to which the candidate drug has not been added before the culturing step of culturing a skin cell culture comprising a dermal fibroblast in a culture medium containing the candidate drug. The preculture may be performed over a discretionary period of time but generally after inoculating the skin cell culture, culturing is performed to subconfluence or confluence. The step of culturing a skin cell culture comprising a dermal fibroblast in a culture medium comprising a candidate drug may be achieved by either adding the candidate drug to the culture medium subjected to the preculturing step or by replacing the culture medium with one containing the candidate drug.

The culturing period for the culturing step of culturing a skin cell culture comprising a dermal fibroblast in a culture medium containing a candidate drug, in view of ensuring sufficient time for the candidate drug to act on the cell, is 24 hours or more, preferably 3 days or more, and more preferably 5 days or more. After the culturing step of culturing a skin cell culture comprising a dermal fibroblast in a culture medium containing a candidate drug, a post-culturing step of further culturing the skin cell culture comprising dermal fibroblasts in a culture medium to which the candidate drug has not been added may be performed.

The culture medium may be any solution in which the cells can survive. Any culture medium that is usually used for culturing a skin cell culture can be used. For example, DMEM, EMEM, and RPMI1640 may be used. Normal culture conditions for culturing a skin cell culture can be used as the culture conditions. For example, culturing may be carried out in 5% CO₂, at 37 °C, in a humidified atmosphere.

The dermal senescence factor is a factor mainly secreted from senescent cells and is a factor that acts on normal dermal cells to inhibit the production of dermal components or promote the degradation thereof. Examples of dermal senescence factor include IGFBP7, CFD, and RSPO4, and anti-aging substances can be screened for by using the expression of one, two, or all of such factors as an indicator.

The step of measuring the expression of a dermal senescence factor in a culture refers to a step of measuring the gene expression level or protein level of the dermal senescence factor in the culture medium by any technique. The level of expression of the senescence factor can be appropriately measured by using a molecular biological technique. For example, when measuring the level of protein, a specific antibody is prepared against each dermal senesce factor and the protein level can be measured by using a technique such as western blot or ELISA. The gene expression levels of dermal senescence factors can be measured by performing RT-PCR using the following primers. The methods are not intended to be limited hereto. A person skilled in the art would be able to appropriately select an internal standard.

**[Table 1]**

| | | |
|---|---|---|
| CFD forward | CATCTGGTTGGTCTTTATTGAGC | (SEQ ID NO: 9) |
| CFD reverse | CATGCTGATCTCGAACTCCTG | (SEQ ID NO: 10) |
| IGFBP7 forward | TCAAAGGACAGAACTCCTGCGTGGTGA | (SEQ ID NO: 11) |
| IGFBP7 reverse | GCTGAAGCCTGTCCTTGGGAATTGGATG | (SEQ ID NO: 12) |
| RSP04 forward | CCAGCAGAGGCTCTTCCTGTTCATCC | (SEQ ID NO: 13) |
| RSP04 reverse | TCCTGGCTGAAGCAGCTCTGACAAG | (SEQ ID NO: 14) |

The step for selecting a candidate drug that can inhibit the expression of a dermal senescence factor is a step for selecting a candidate drug as an anti-aging factor if the expression of the dermal senescence factor is reduced. For comparison, the expression of the dermal senescence factor in a culture is measured for a control group which differs only in that the candidate drug is not added. The measurement results of the control group can be compared. Experiments involving the control group can be performed at the same time as the screening method of the present invention, or the experiments involving the control group can be performed beforehand and just the results used for comparison. In another embodiment, the expression levels of a dermal senescence factor before and after the addition of a candidate drug are compared and if the expression level decreases, the candidate drug can be selected as an anti-aging factor. The expression of a dermal senescence factor is only required to decrease compared to the control group but may decrease with a significant difference.

The skin cell culture may be a culture of any cells that constitute the skin. Cells that constitute the skin may include epidermal keratinocytes, dermal fibroblasts, adipocytes present in the subcutaneous fat layer, and other cells such as Langerhans cells, melanocytes, Merkel cells, histocytes, mast cells, plasma cells, and stem cells, and may also contain cells that make up each organ in the skin, such as sebaceous glands, sweat glands, and hair follicles. Dermal fibroblasts may be cultured alone or co-cultured with other cells. As one embodiment for a co-culture, dermal fibroblasts and epidermal cells are co-cultured to construct a 3-dimensional model skin culture, and a candidate drug may be added thereto. In another embodiment, a culture in which dermal fibroblasts and stem cells are co-cultured may be used. The stem cells may be epidermal stem cells present in the epidermis or mesenchymal stem cells present in the dermis or subcutaneous adipose tissue.

Dermal fibroblasts are cells that belong to the mesenchymal group of cells and produce collagen fibers, elastic fibers, and mucopolysaccharides. Fibroblasts have a relatively high rate of division and by repeated passaging, senescent fibroblasts can be obtained.

Mesenchymal stem cells generally refer to stem cells derived from any tissue classified as mesenchymal. Tissue classified as mesenchymal include bone marrow, placenta, adipose tissue, dermis, etc. Examples of stem cells derived from these tissues include bone marrow-derived mesenchymal stem cells (hereinafter simply referred to as bone marrow stem cells), placenta-derived mesenchymal stem cells, adipose tissue-derived mesenchymal stem cells (hereinafter simply referred to as adipose stem cells), dental pulp-derived mesenchymal stem cells, umbilical cord blood-derived mesenchymal stem cells, dermis-derived mesenchymal stem cells (hereinafter simply referred to as dermal stem cells). Some dermal papilla cells are also considered to be mesenchymal stem cells.

Bone marrow-derived mesenchymal stem cells are stem cells present in the bone marrow. Bone marrow stem cells have the capacity to differentiate into various tissues including cardiomyocytes, skeletal muscle cells, vascular endothelial cells, chondrocytes, osteocytes, and adipocytes. Bone marrow-derived mesenchymal stem cells are characterized by CD105, CD73, CD90 being positive markers, but are not intended to be limited to cells that express these markers. Bone marrow mesenchymal cells are released into the blood in response to signals from tissue damage and the like and are considered to perform a function of healing the wound.

Adipose tissue-derived mesenchymal stem cells can be simply referred to as adipose stem cells and are a type of mesenchymal stem cell present in the adipose tissue. It is known that adipose stem cells have the capacity to migrate (NPL 3 and PL 2) and it is thought that migration occurs through the adipose tissue. Adipose stem cells are characterized by the expression of CD90 markers, CD105 markers, etc., but this is not intended to limit the cells to those expressing these markers.

Dermis derived mesenchymal stem cells can be simply referred to as dermal stem cells and are stem cells that are present in the dermis. Dermal stem cells contribute to the maintenance of dermal layer homeostasis by replenishing fibroblasts by differentiating into fibroblasts or through the activation of fibroblasts. Dermal mesenchymal stem cells are present primarily in the dermal layer as a pericyte-like cell group around microvascular sites or organs such as sebaceous glands, sweat glands, and hair follicles. It is thought that dermal mesenchymal stem cells are activated when the skin is damaged, differentiating into fibroblasts or myofibroblasts and contributing to the regeneration and repair of the skin. Dermal stem cells are characterized by being double positive for CD34 and NG2, but this is not intended to limit the cells to those expressing these markers. It is thought that dermal stem cells are replenished through blood vessels since capillaries are spread over the vicinity of each organ in which dermal stem cells are present. Thus, dermal stem cells can have properties similar to bone-marrow mesenchymal stem cells.

The cells to be used are preferably senescent cells since it is necessary for a senescence factor to be produced before adding a candidate drug. For the senescent cells, cells having a decreased proliferation rate (e.g. a doubling time of less than 0.5/week and Pdl > 50) after subculturing for a fixed period, for example, 3 months or more, are preferably used. Alternatively, primary cells can be acquired from elderly people to obtain senescent cells. In a preferred embodiment, the use of the thus-obtained senescent cells in which the expression of a senescent factor selected from the group consisting of IGFBP7, CFD, and RSPO4 has been confirmed is preferable. The senescent cells can be any cells provided they are contained in the skin, but dermal fibroblasts are preferable in view of expressing a senescence factor selected from the group consisting of IGFBP7, CFD, and RSPO4.

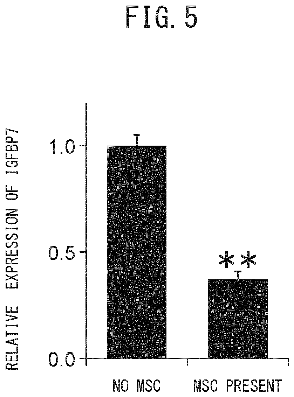

When the skin cell culture contains only dermal fibroblasts, the anti-aging substance selected by the screening method can inhibit the expression of the senescence factor by directly acting on senescent dermal fibroblasts. Meanwhile, when the skin cell culture is a co-culture of dermal fibroblasts and another type of cell, the anti-aging substance selected by the screening method can inhibit the expression of the senescence factor by either directly acting on senescent dermal fibroblasts or indirectly acting thereon through the other co-cultured cells. It is thought that for the indirect action, senescence factor expression in senescent dermal fibroblasts is suppressed through, for example, the activity of the co-cultured cells. It has been shown that mesenchymal stem cells can inhibit the expression of a senescence factor (IGFBP7) by acting indirectly on dermal fibroblasts (FIG. 5).

When performing a co-culture, dermal fibroblasts and other cells may be mixed and inoculated on the same place, layered and cultured, or isolated and cultured in the same culture vessel. When isolating and culturing cells in the same vessel, exchange of the culture medium is possible, and by culture medium being mixed through pores of a size sufficient to prevent the migration of cells therethrough, a factor secreted by one group of cells can affect the other group of cells. In one embodiment, stem cells can be co-cultured with dermal fibroblasts. Such stem cells have the capacity to differentiate into dermal fibroblasts as well as the capacity to act on surrounding cells by releasing some sort of factor. Thus, by using this co-culturing system, it is possible to screen for an anti-aging substance that can act on stem cells and inhibit senescent dermal fibroblasts from producing a senescence factor.

Therefore, the anti-aging substance selected by the screening method of the present invention may be one that acts directly on senescent cells to inhibit secretion of a senescent factor or one that acts on another type of cell, such as stem cells, to indirectly inhibit secretion of a senescence factor, depending on the system used. Anti-aging substances that act directly on senescent cells may be referred to as senescence factor expression inhibitors. Senescence factors inhibit production of dermal components or promote degradation thereof. Thus, inhibition of senescence factor expression has the effect of increasing dermal components such as collagen or elastin. Therefore, anti-aging substances can be blended with cosmetics or pharmaceutical agents to be used as agents for promoting an increase in dermal components, and as agents for ameliorating wrinkles and sagging, and as an anti-aging agent. Anti-aging substances that act on stem cells and indirectly inhibit the secretion of aging factors can be used as stem cell activators and may be used alone. However, the combined use thereof with stem cell therapy or the administration thereof in combination with a stem cell attractant can bring about even more desirable anti-aging effects.

When the screening method of the present invention was carried out using a plurality of cosmetic materials as candidate drugs, a turmeric extract and a Rosa multiflora extract could be screened for as anti-aging factor expression inhibitors.

The turmeric extract refers to an extract from a perennial plant of the ginger family Curcuma Longa which is native to India. Parts of the plant which are extracted include the whole plant, the leaves, the flower, and the roots, but the rhizome is particularly preferable. Any extraction method can be used for the extraction method but, for example, an extract can be obtained by performing steam distillation or solvent extraction on dried roots. Further, any extraction solvent can be used as a solvent but a 50% butylene glycol or the like can be used.

The Rosa multiflora extract refers to an extract of the fruit or false fruit of Rosa multiflora which is native to east Asia. Any extraction method may be used but, for example, an extract can be obtained by performing steam distillation or solvent extraction on fruit or false fruit that have been dried. Further, any solvent may be used as the solvent, but an aqueous ethanol solution or 50% butylene glycol or the like can be used.

The candidate drug may be a drug belonging to any library. The library may be an organic compound library, an extract library, a cosmetic material library, a pharmaceutical library, etc. From the viewpoint of transdermal administration of the candidate drug, it is preferable to use, as the candidate drug, a cosmetic material that has already been shown to be stable in transdermal administration.

Anti-aging substances screened for by the method of the present invention can be blended in any composition, for example, cosmetics, pharmaceuticals, and quasi drugs. The cosmetics, pharmaceuticals, or quasi-drugs into which the anti-aging substances have been blended are used on subjects worried about skin aging such as wrinkles and sagging. Such subjects who are worried about skin aging could be considered to have high expression of senescence factors in the skin. In particular, in the field of beauty, since anti-aging is desirable, it is preferable that the substance be blended in cosmetics. The dosage form of the cosmetic in which the substance is blended is discretionary and the substance may be blended in the form of a liquid, gel, foam, emulsion, cream, ointment, sheet, etc. The anti-aging substance can be applied to various products by being blended in such cosmetic products as: basic cosmetics such as pharmaceutical preparations for external use, toners, emulsions, creams, ointments, lotions, oils, packs; makeup cosmetics such as facial cleansers and skin cleansers, hair removers, depilatory agents, after shave lotions, pre shave lotions, shaving cream, foundation, lipstick, blusher, eye shadow, eyeliner, mascara; hair care products such as shampoo, conditioner, hair treatment, pre-hair treatment, hair styling products, perm agents, hair tonic, hair dye, hair growth and nourishment product; and bath preparations.

In the present invention, IGFBP7, CFD, and RSO4, have been determined to be senescence factors. Thus, by examining the expression of these factors, the degree of senescence of a cell can be determined. For example, by measuring the level of expression of a gene as an internal standard, the degree of senescence of a cell can be determined. The degree of senescence can be determined for a cell obtained from a living body, in particular a skin cell, and can also be determined for cultured cells. Thus, in yet a further embodiment of the present invention, the present invention relates to a method of determining the degree of senescence of a cell using the level of expression of senescence factors as an indicator.

All documents mentioned herein are incorporated herein by reference in their entirety.

The embodiments of the present invention described below are for illustrative purposes only and do not limit the technical scope of the present invention. The technical scope of the present invention is limited only by the claims. Modifications of the present invention, for example, additions, deletions and replacements of the constituent features can be made without departing from the spirit of the present invention.

### EXAMPLE 1: Effect of senescent cells on normal cells

### Cell Culture

Dermal fibroblasts established from skin from the back of an adult (21 year old) were cultured in Dulbecco's Modified Eagle Medium supplemented with 10% FBS. Cells with a culture period of less than 1 month and Pdl < 30 were used as normal fibroblasts (young fibroblasts). These cells were cultured for 3 months or more and cells which began to proliferate more slowly (doubling time less than 0.5/week and Pdl > 50) were used as senescent cells. Normal fibroblasts were inoculated on the upper layer of a 6-well transwell system (Falcon; Franklin Lakes, NJ) filled with Dulbecco's Modified Eagle Medium supplemented with 10% FBS until the density was 1250 cell/cm². Subconstructed senescent fibroblasts and normal fibroblasts were separately inoculated in two wells of the lower layer of the transwell system. The upper layer and the lower layer of the transwell system were combined and cultured for 2 days at 37 °C in a humidified atmosphere with 5% CO₂, and the cells in the upper layer and the lower layer were recovered using Qiazole (Qiagen).

### Quantitative RT-PCR

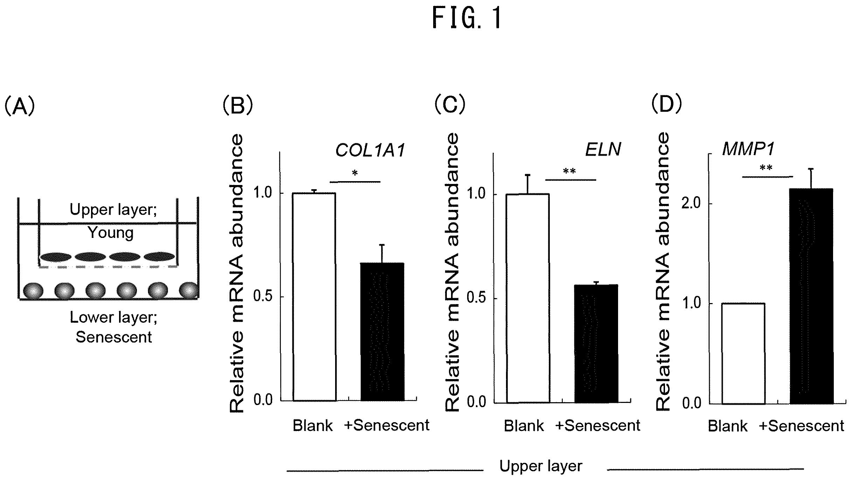

RNA was extracted from the recovered cell lysate using RNeasy Protect Kit (Qiagen) by following the instructions therefor, then Superscript VILO (Invitrogen) was used to reverse transcribe the RNA into cDNA by following the instructions. Using 28SrRNA as an internal standard, real time PCR was performed with LightCycler (commercially available) using the primer pairs for the following genes. The results are shown in FIG. 1. The cycle threshold (Ct value) calculated by Lightcycler software ver. 3.5 was normalized for GAPDH mRNA of each sample.

**[Table 2]**

| | | |
|---|---|---|
| *MMP1* forward | ATTTGCCGACAGAGATGAAGTCC | (SEQ ID NO: 1) |
| *MMP1* reverse | GGGTATCCGTGTAGCACATTCTG | (SEQ ID NO: 2) |
| *ELN* forward | TGTCCATCCTCCACCCCTCT | (SEQ ID NO: 3) |
| *ELN* reverse | CCAGGAACTCCACCAGGAAT | (SEQ ID NO: 4) |
| *C0L1A1* forward | AGCAGGCAAACCTGGTGAAC | (SEQ ID NO: 5) |
| *C0L1A1*reverse | AACCTCTCTCGCCTCTTGCT | (SEQ ID NO: 6) |
| *GAPDH* forward | GAAGGTGAAGGTCGGAGT | (SEQ ID NO: 7) |
| *GAPDH* reverse | GAAGATGGTGATGGGTTTC | (SEQ ID NO: 8) |

As illustrated in FIG. 1 (A), when senescent fibroblasts were inoculated on the lower layer, the expression of MMP1 was significantly increased compared to when the cells were not inoculated on the bottom layer. Furthermore, the expression of elastin and collagen was significantly decreased. When normal fibroblasts (young fibroblasts) were inoculated on the lower layer, there was no significant change compared to when cells were not inoculated on the bottom layer (not shown). From these results, it was clear that the senescent cells secreted some senescence factor, whereby gene expression was changed in dermal fibroblasts sharing the culture medium therewith.

### Identification of genes with increased expression in senescent fibroblasts

The difference in gene expression between young fibroblasts and senescent fibroblasts was examined by microarray analysis. Genes expressed at least 2.5 times more in senescent fibroblasts than young fibroblasts were identified. The genes listed in the table below were identified as senescence factor candidates.

**[Table 3]**

| Gene Name | Description | Systematic Name | Fold Increased |
|---|---|---|---|
| RSP04 | Homo sapiens R-spondin family, member 4 | NM_001029871 | 5.9 |
| IGFBP7 | Homo sapiens insulin-like growth factor binding protein 7 | NM_001553 | 3.6 |
| CFD | Homo sapiens complement factor D | NM_001928 | 2.7 |

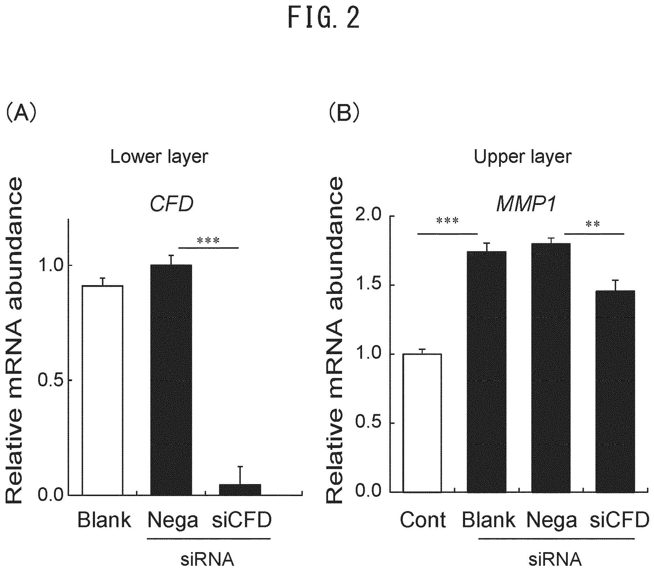

Of these senescence factor candidate genes, siRNAs were designed to knockdown CFD (SI00030100 or SI00030107 (Qiagen), both have similar inhibitory effects) and an siRNA (SI030640318 (Qiagen)) was used as a negative control. The siRNAs were introduced into the senescent fibroblasts and, following the aforementioned cell culture protocol, cells with the CFD gene knocked down were inoculated onto the lower layer of the transwell system and cultured together with the cells of the upper layer for 2 days. The cells of the lower layer and the cells of the upper layer were separately acquired and the expression of CFD in the cells of the lower layer and the expression of MMP1 in the cells of the upper layer were examined. When senescent fibroblasts with knocked down CFD were inoculated on the lower layer, the expression of MMP1 from cells in the upper layer decreased. The results are shown in FIG. 2. These results show that CFD was identified as a senescence factor.

### Expression of senescence factors in senescent cells

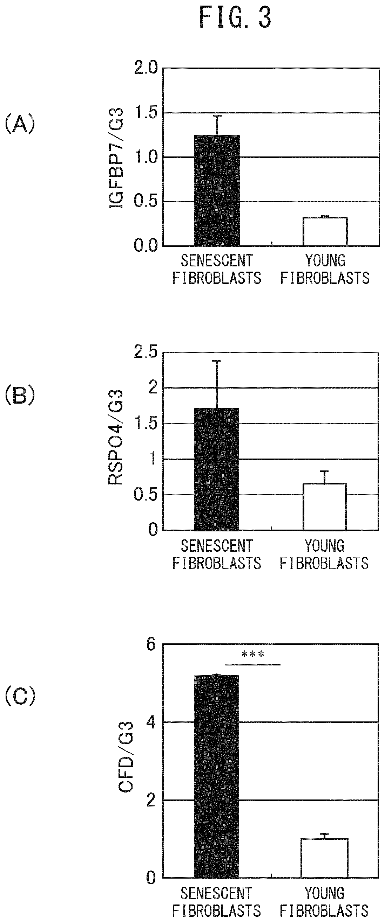

The expression of the senescence factor (CFD) identified by the present inventors and candidate senescence factors (IGFBP7 and RSPO4) were compared between senescent fibroblasts and young fibroblasts. Senescent and young fibroblasts were separately cultured then recovered using Qiazole (Qiagen). The recovered cell lysates were examined for expression of senescence factors using the same method as the aforementioned RT-PCR. The following primer pairs were used. The results are shown in FIG. 3. It was shown that the senescence factor identified in the present invention and the candidate senescence factors were highly expressed by senescent fibroblasts.

**[Table 4]**

| | | |
|---|---|---|
| CFD forward | CATCTGGTTGGTCTTTATTGAGC | (SEQ ID NO: 9) |
| CFD reverse | CATGCTGATCTCGAACTCCTG | (SEQ ID NO: 10) |
| IGFBP7 forward | TCAAAGGACAGAACTCCTGCCTGGTGA | (SEQ ID NO: 11) |
| IGFBP7 reverse | GCTGAAGCCTGTCCTTGGGAATTGGATG | (SEQ ID NO: 12) |
| RSP04 forward | CCAGCAGAGGCTCTTCCTGTTCATCC | (SEQ ID NO: 13) |
| RSP04 reverse | TCCTGGCTGAAGCAGCTCTCACAAG | (SEQ ID NO: 14) |
| *G APDH* forward | GAAGGTGAAGGTCGGAGT | (SEQ ID NO: 7) |
| *GAPDH* reverse | GAAGATGGTGATGGGATTTC | (SEQ ID NO: 8) |

### Addition of senescence factor

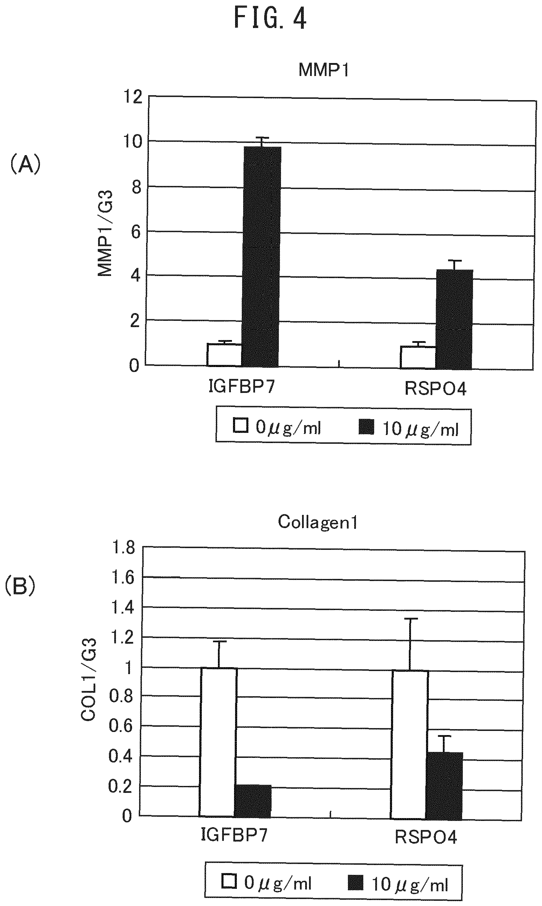

The proteins of the candidate senescence factors (IGFBP7 and RSPO4) (purchased from Serotec, R&D SYSTEMS) were added to the culture medium for culturing the young fibroblasts at a concentration of 0 µg/ml and 10 µg/ml. After culturing for 2 days, the cells were then separately recovered using Qiazole (Qiagen). In the same way as disclosed in the section relating to quantitative RT-PCR, the expression levels of MMP1 and collagen were examined in the recovered cell lysate. The results are shown in FIG. 4. IGFBP7 and RSPO4 reduced expression of collagen and also increased MMP1 expression. Accordingly, this shows that IGFBP7 and RSPO4 also act as senescence factors.

### EXAMPLE 2: Inhibition of senescence factors by mesenchymal stem cells

### Cell culture

Senescent fibroblasts were inoculated on the upper layer of a 6-well transwell system (Falcon; Franklin Lakes, NJ) filled with Dulbecco's Modified Eagle Medium supplemented with 10% FBS until the density was 1250 cell/cm². Mesenchymal stem cells (MSCs) were purchased from Lonza and cultured on MessenPro culture medium. Subconstructed MSCs were inoculated on the lower layer well of the transwell system. 24 hours later, the culture medium was exchanged with DMEM comprising 10% FBS and 250 µM acorbic acid, and co-culturing was started. Two days later, Qiazole (Qiagen) was used and the cells were recovered. The recovered cell lysate was examined for gene expression of senescence factor (IGFBP7) using the same method as for the aforementioned RT-PCR. The results are shown in FIG. 5. As a control group, fibroblasts were cultured alone with no MSCs. In the presence of MSCs, gene expression of IGFBP7 was significantly reduced (student's t-test: P < 0.05).

### EXAMPLE 3: Screening method using senescence factor as an indicator

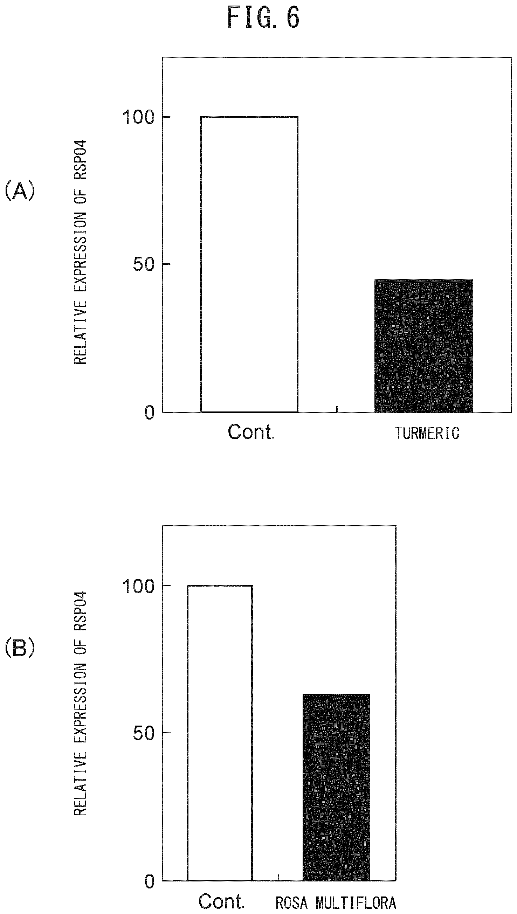

Senescent fibroblasts were inoculated in a 24-well plate such that the density thereof would be 2500 cells/cm². 24 hours after inoculation, added thereto was a 0.5% concentration of each of the following extracts which are cosmetic materials: a Rosa multiflora extract and a turmeric extract. A group in which only the extraction solvent for the extract was added served as a control. 24 hours after adding the extracts, the cells were recovered using Qiazol (Qiagen). The expression levels of RSPO4 were examined in the recovered cell lysate using the same method as described in the section relating to quantitative RT-PCR. RSPO4 expression in the control group was set as 100 and RSPO4 expression in the addition group was shown. The results are shown in FIG. 6.

## Claims

1. A screening method for an anti-aging substance using the expression of a dermal senescence factor as an indicator.

2. The screening method according to claim 1, comprising the steps of:
culturing a skin cell culture comprising a dermal fibroblast in a culture medium comprising a candidate drug;
measuring expression of the dermal senescence factor in the culture; and
selecting a candidate drug that can inhibit the expression of the dermal senescence factor.

3. The screening method according to claim 2, wherein the skin cell culture further comprises a stem cell.

4. The screening method according to any one of claims 1 to 3, wherein the expression of the dermal senescence factor is determined by measuring the level of gene or protein expression of the dermal senescence factor.

5. The screening method according to any one of claims 1 to 4, wherein the dermal senescence factor is IGFBP7, CFD, or RSPO4.

6. A dermal senescence factor expression inhibitor comprising a turmeric extract or a Rosa multiflora extract.

7. The dermal senescence factor expression inhibitor according to claim 6, wherein the dermal senescence factor is CFD.

8. An aesthetic or non-therapeutic method of ameliorating wrinkles, sagging, or skin aging, comprising inhibiting expression of a dermal senescence factor.

9. The aesthetic or non-therapeutic method according to claim 8, wherein the dermal senescence factor is IGFBP7, CFD, or RSPO4.


SEQUENCE LISTING

<110>  Shiseido Co., Ltd.

<120>  Method for Screening an Anti-Aging Material

<130>  P180115WO

<150>  JP2017-139181
<151>  2017-07-18

<160>  14

<170>  PatentIn version 3.5

<210>  1
<211>  23
<212>  DNA
<213>  Artificial Sequence

<220>
<223>  MMP1 forward Primer

<400>  1
atttgccgac agagatgaag tcc                                              23


<210>  2
<211>  23
<212>  DNA
<213>  Artificial Sequence

<220>
<223>  MMP1 reverse  Primer

<400>  2
gggtatccgt gtagcacatt ctg                                              23


<210>  3
<211>  20
<212>  DNA
<213>  Artificial Sequence

<220>
<223>  ELN forward Primer

<400>  3
tgtccatcct ccacccctct                                                  20


<210>  4
<211>  20
<212>  DNA
<213>  Artificial Sequence

<220>
<223>  ELN reverse Primer

<400>  4
ccaggaactc caccaggaat                                                  20

<210>  5
<211>  20
<212>  DNA
<213>  Artificial Sequence

<220>
<223>  COL1A1 forward Primer

<400>  5
agcaggcaaa cctggtgaac                                                    20


<210>  6
<211>  20
<212>  DNA
<213>  Artificial Sequence

<220>
<223>  COL1A1 reverse Primer

<400>  6
aacctctctc gcctcttgct                                                    20


<210>  7
<211>  18
<212>  DNA
<213>  Artificial Sequence

<220>
<223>  GAPDH forward Primer

<400>  7
gaaggtgaag gtcggagt                                                      18


<210>  8
<211>  20
<212>  DNA
<213>  Artificial Sequence

<220>
<223>  GAPDH reverse Primer

<400>  8
gaagatggtg atgggatttc                                                    20


<210>  9
<211>  23
<212>  DNA
<213>  Artificial Sequence

<220>
<223>  CFD forward Primer

<400>  9
catctggttg gtctttattg agc                                                23


<210>  10
<211>  21
<212>  DNA

<213>  Artificial Sequence

<220>
<223>  CFD reverse Primer

<400>  10
catgctgatc tcgaactcct g                                                21


<210>  11
<211>  27
<212>  DNA
<213>  Artificial Sequence

<220>
<223>  IGFBP7 forward Primer

<400>  11
tcaaaggaca gaactcctgc ctggtga                                          27


<210>  12
<211>  28
<212>  DNA
<213>  Artificial Sequence

<220>
<223>  IGFBP7  reverse Primer

<400>  12
gctgaagcct gtccttggga attggatg                                         28


<210>  13
<211>  26
<212>  DNA
<213>  Artificial Sequence

<220>
<223>  RSPO4 forward Primer

<400>  13
ccagcagagg ctcttcctgt tcatcc                                           26


<210>  14
<211>  25
<212>  DNA
<213>  Artificial Sequence

<220>
<223>  RSPO4 reverse Primer

<400>  14
tcctggctga agcagctctc acaag                                            25